# EUROPÄISCHE PATENTSCHRIFT

(11) **EP 1 954 203 B1**
(45) Veröffentlichungstag und Bekanntmachung des Hinweises auf die Patenterteilung: **02.10.2013**
(21) Anmeldenummer: 06819409.1
(22) Anmeldetag: 11.11.2006
(51) Int. Cl.: A61B 17/17

(54) **CHIRURGISCHES FÜHRUNGSINSTRUMENT**
SURGICAL GUIDE INSTRUMENT
INSTRUMENT CHIRURGICAL DE GUIDAGE

(30) Priorität: 24.11.2005 DE 102005056818
(43) Veröffentlichungstag der Anmeldung: 13.08.2008
(73) Patentinhaber: Aesculap AG, 78532 Tuttlingen (DE)
(72) Erfinder: SCHNEID, Susanne, 89233 Neu-Ulm (DE); FISCHER, Kay, 78532 Tuttlingen (DE); MATTES, Uwe, 78532 Tuttlingen (DE)
(74) Vertreter: Hoeger, Stellrecht & Partner Patentanwälte
(86) Internationale Anmeldenummer: PCT/EP2006/068367
(87) Internationale Veröffentlichungsnummer: WO 2007/060099

(56) Entgegenhaltungen:
- WO-A2-02/11633
- WO-A2-2004/019785
- US-A1- 2004 002 711
- US-A1- 2004 143 332

## Beschreibung

Die vorliegende Erfindung betrifft ein chirurgisches Führungsinstrument für ein chirurgisches, zum Bearbeiten von Wirbelkörpern ausgebildetes und ein distales Werkzeugende aufweisendes Bearbeitungswerkzeug, wobei das Führungsinstrument einen im Wesentlichen langgestreckten, ein proximales und ein distales Ende aufweisenden und eine Längsachse definierenden Instrumentenkörper umfasst, der eine Führungsvorrichtung für das Bearbeitungswerkzeug trägt, wobei die Führungsvorrichtung derart angeordnet und ausgebildet ist, dass mit ihr das Bearbeitungswerkzeug entlang einer von der Führungsvorrichtung definierten Bewegungsbahn zwangsführbar ist und dass die Bewegungsbahn einer überlagerten Translations-Schwenk-Bewegung entspricht.

Chirurgische Führungsinstrumente der eingangs beschriebenen Art werden beispielsweise dazu verwendet, chirurgische Meißel oder chirurgische Fräswerkzeuge zu führen, um Knochen oder Knochenteile eines menschlichen oder tierischen Körpers zu bearbeiten. Sie dienen in erster Linie dem Zweck, zu vermeiden, dass das Bearbeitungswerkzeug von einem Operateur frei gehalten werden muss. Eine solche Vorgehensweise birgt insbesondere Nachteile, wenn Operationen im Bereich der Wirbelsäule vorgenommen werden, da dabei ein hohes Risiko besteht, im oder aus dem Spinalkanal heraus verlaufende Nervenbahnen zu verletzen.

Auf Grund ihrer Bauform und Baugröße eignen sich bekannte chirurgische Führungsvorrichtungen nicht zum Führen chirurgischer Bearbeitungswerkzeuge bei minimalinvasiven Eingriffen. Dies insbesondere auch deshalb, weil durch das Führungsinstrument und das Bearbeitungswerkzeug die Sicht auf das Operationsfeld stark eingeschränkt ist.

Aus der WO 2004/019785 A2 ist eine Vorrichtung zur Bearbeitung von Teilen bekannt. In der WO 02/11633 sind Verfahren und Vorrichtungen für stereotaktische Implantationen offenbart.

Es ist daher Aufgabe der vorliegenden Erfindung, ein chirurgisches Führungsinstrument der eingangs beschriebenen Art so zu verbessern, dass es insbesondere bei minimalinvasiven chirurgischen Eingriffen einsetzbar ist.

Diese Aufgabe wird bei einem chirurgischen Führungsinstrument der eingangs beschriebenen Art erfindungsgemäß dadurch gelöst, dass die Führungsvorrichtung ein erstes Führungselement umfasst, welches eine Schwenkachse für das Bearbeitungswerkzeug definiert, so dass das Bearbeitungswerkzeug relativ zum ersten Führungselement verschiebbar und um die Schwenkachse verschwenkbar ist.

Die erfindungsgemäße Weiterbildung bekannter chirurgischer Führungsinstrumente hat den Vorteil, dass das Bearbeitungswerkzeug durch die speziell ausgebildete Führungsvorrichtung mindestens teilweise aus dem Operationsfeld heraus verschwenkt werden kann, so dass eine Sicht auf das Operationsfeld für einen Operateur deutlich verbessert ist. Insbesondere eröffnet eine solche Führungsvorrichtung die Möglichkeit, das Bearbeitungswerkzeug so zu führen, dass, falls dieses einen Griffteil aufweist, dieser praktisch während des Gesamtbearbeitungsvorgangs außerhalb des Operationsfeldes und des Sichtbereichs auf das Operationsfeld bewegbar ist. Gemäß der Erfindung ist vorgesehen, dass die Führungsvorrichtung ein erstes Führungselement umfasst, welches eine Schwenkachse für das Bearbeitungswerkzeug definiert, so dass das Bearbeitungswerkzeug relativ zum ersten Führungselement verschiebbar und um die Schwenkachse verschwenkbar ist. Unabhängig davon, wie die Führungsvorrichtung im Übrigen ausgebildet ist, kann mit dem ersten Führungselement insbesondere eine überlagerte Translations-Schwenk-Bewegung für das Bearbeitungswerkzeug vorgegeben werden. Vorzugsweise definiert das erste Führungselement die Schwenkachse.

Damit beispielsweise mit dem Bearbeitungswerkzeug geradlinige Nuten oder Rücksprünge präpariert werden können, ist es günstig, wenn die Führungsvorrichtung derart angeordnet und ausgebildet ist, dass mit ihr eine Spitze oder ein Zentrum des distalen Werkzeugendes entlang einer linearen Bahnkurve zwangsführbar ist. Dies bedeutet, dass beispielsweise eine kugelige rotierende Spitze eines Fräswerkzeugs mit ihrem Zentrum entlang einer Geraden oder eines geraden Abschnitts bewegbar ist, wobei gleichzeitig das Bearbeitungswerkzeug sowohl eine Translationsbewegung als auch eine Schwenkbewegung ausführt. Selbstverständlich kann die Führungsvorrichtung jedoch auch so ausgebildet sein, dass eine Spitze oder ein Zentrum des distalen Werkzeugendes entlang einer beliebigen, insbesondere ein- oder mehrfach gekrümmten Bahnkurve zwangsführbar ist.

Damit ein Operateur das Bearbeitungswerkzeug nicht während des gesamten chirurgischen Eingriffs halten muss, ist es vorteilhaft, wenn die Führungsvorrichtung eine Halterung für das Bearbeitungswerkzeug umfasst.

Grundsätzlich wäre es denkbar, die Halterung als Teil des Bearbeitungswerkzeugs auszubilden. Damit jedoch sowohl das Führungsinstrument als auch das Bearbeitungswerkzeug optimal reinigbar sind, ist es günstig, wenn die Halterung mit dem Bearbeitungswerkzeug lösbar verbindbar ist.

Ein besonders einfacher Aufbau des Führungsinstruments ergibt sich, wenn die Halterung eine Klemmeinrichtung zum klemmenden Verbinden mit einem Bearbeitungswerkzeug umfasst. Beispielsweise kann die Klemmeinrichtung zwei Klemmbacken aufweisen, zwischen die das Bearbeitungswerkzeug einspannbar ist. Alternativ wäre auch eine Klemmung mittels eines rohrschellenartigen Bauteils möglich.

Die Handhabung des Führungsinstruments und eines an diesem geführten Bearbeitungswerkzeugs wird weiter vereinfacht, wenn das Bearbeitungswerkzeug oder ein Teil desselben am ersten Führungselement verschiebbar und verschwenkbar lagerbar ist.

Eine optimale Führung bei gleichzeitig minimaler Baugröße des Führungsinstruments kann dadurch erreicht werden, dass das erste Führungselement am distalen Ende oder im Bereich des distalen Endes des Instrumentenkörpers angeordnet ist.

Um auf einfache Weise mit dem ersten Führungselement eine Schwenkbewegung zu definieren, weist das erste Führungselement günstigerweise eine bezogen auf eine die Längsachse enthaltende Ebene konvex gekrümmte Führungsfläche auf. Dies gestattet es, einen Teil des Bearbeitungswerkzeugs in Anlage an die Führungsfläche sowohl an dieser abgleiten als auch an dieser abrollen zu lassen. Bei Kombination beider Bewegungen ergibt sich eine überlagerte Translations-Schwenk-Bewegung des Bearbeitungswerkzeugs.

Um eine optimale Führung in einer die Längsachse schneidenden Ebene zu erreichen, ist vorteilhafterweise die Führungsfläche in der die Längsachse schneidenden Ebene in Richtung auf die Längsachse hin konkav gekrümmt.

Eine besonders gute Zwangsführung für das Bearbeitungswerkzeug lässt sich dadurch erreichen, dass das erste Führungselement eine Führungshülse mit einer Führungsdurchbrechung für das Bearbeitungswerkzeug oder einen Teil, insbesondere einen Schaft, desselben umfasst. Das Bearbeitungswerkzeug kann durch die Führungshülse eine Translationsbewegung und/oder eine Schwenkbewegung zwangsgeführt ausführen.

Der Aufbau des Führungsinstruments vereinfacht sich weiter, wenn die Führungsdurchbrechung eine innere Wandfläche aufweist und wenn die innere Wandfläche die Führungsfläche bildet. Insbesondere kann die Führungsfläche in Richtung auf eine Längsachse der Führungsdurchbrechung hin weisend konvex gekrümmt sein.

Die Stabilität des Führungsinstruments wird weiter verbessert, wenn die Führungsvorrichtung ein zweites Führungselement zum Halten und Führen des Bearbeitungswerkzeugs am Instrumentenkörper umfasst und wenn das Bearbeitungswerkzeug relativ zum zweiten Führungselement verschiebbar und verschwenkbar lagerbar ist. Das erste und das zweite Führungselement zusammen bilden eine optimale, für einen Operateur leicht handhabbare Führungsvorrichtung für ein Bearbeitungswerkzeug.

Dam it ein Operateur das Bearbeitungswerkzeug besonders einfach unter Zuhilfenahme des Führungsinstruments bewegen kann, ist das Bearbeitungswerkzeug oder ein Teil desselben am zweiten Führungselement verschiebbar und um die Schwenkachse verschwenkbar lagerbar.

Eine hohe Präzision bei der Führung des Bearbeitungswerkzeugs lässt sich dadurch erreichen, dass das zweite Führungselement am proximalen Ende oder im Bereich des proximalen Endes des Instrumentenkörpers angeordnet ist. Insbesondere ist es wünschenswert, dass ein Abstand der beiden Führungselemente voneinander maximal groß ist.

Auf besonders einfache Weise kann das Bearbeitungswerkzeug mit dem Führungsinstrument geführt werden, wenn das zweite Führungselement mindestens ein erstes Führungsglied umfasst zum Führen des Bearbeitungswerkzeugs oder eines Teils desselben längs einer durch das zweite Führungselement definierten Kurve. Beispielsweise kann das Führungsglied in Form einer Vertiefung oder eines Vorsprungs ausgebildet sein.

Besonders vorteilhaft ist es, wenn das zweite Führungselement mindestens einen Führungskörper umfasst und wenn das mindestens eine erste Führungsglied in Form einer am mindestens einen Führungskörper angeordneten Führungsnut ausgebildet ist. Beispielsweise kann so ein Vorsprung am Bearbeitungswerkzeug oder an einer Halterung für dieses in der Führungsnut geführt werden. Die Führungsnut selbst kann geradlinig oder gekrümmt ausgebildet sein, so dass auf Grund der Form und vorgebbaren Richtung der Führungsnut eine Translations-Schwenk-Bewegung für das Bearbeitungswerkzeug auf einfache Weise vorgebbar ist.

Noch einfacher wird der Aufbau des Führungsinstruments, wenn zwei symmetrisch angeordnete Führungskörper vorgesehen sind. Beispielsweise können diese so ausgebildet sein, dass an diesen vorgesehene Führungsnuten aufeinander zu weisen, so dass seitlich abstehende Vorsprünge am Bearbeitungswerkzeug oder an einer Halterung für das Bearbeitungswerkzeug in die Führungsnuten eintauchen können. Denkbar wäre es auch, vier Führungskörper vorzusehen, um am Führungsinstrument eine zweite Führungsvorrichtung auszubilden.

Günstigerweise weist der mindestens eine Führungskörper zwei oder mehr Führungsnuten auf. Dies gestattet es einem Operateur, unterschiedliche Translations-Schwenk-Bewegungen mit dem Bearbeitungswerkzeug zwangsgeführt auszuführen, und eröffnet ihm die Möglichkeit, beispielsweise einen Knochen in unterschiedlicher Weise zu bearbeiten, ohne dass er ein weiteres Instrument hierfür benötigen würde.

Vorteilhafterweise ist das mindestens eine erste Führungsglied relativ zur Längsachse geneigt. Beispielsweise kann eine relativ zur Längsachse geneigte Führungsnut verhindern, dass die Sicht auf einen Operationsbereich unnötig eingeschränkt wird.

Günstig ist es, wenn die Halterung mindestens ein zweites Führungsglied umfasst und wenn das mindestens eine zweite Führungsglied derart ausgebildet ist, dass es am ersten Führungsglied bewegbar gelagert ist. Die Halterung derart auszugestalten hat den Vorteil, dass eine sichere Führung beliebiger Bearbeitungswerkzeuge mit Hilfe der Halterung am Führungsinstrument möglich ist.

Ein besonders einfacher Aufbau des Führungsinstruments lässt sich dadurch erreichen, dass das mindestens eine zweite Führungsglied ein quer oder im Wesentlichen quer zur Längsachse abstehender, zum Eintauchen in die mindestens eine Führungsnut geformter Führungsvorsprung ist. Beispielsweise könnte der Führungsvorsprung in Form eines kurzen zylindrischen Zapfens ausgebildet sein. Diese Ausgestaltung ermöglicht es zudem, dass der Führungsvorsprung in einer Führungsnut verschoben und relativ zu dieser auch verschwenkt werden kann.

Günstigerweise ist das zweite Führungselement vom ersten Führungselement in Längsrichtung beabstandet angeordnet. Je größer der Abstand der beiden Führungselemente voneinander, um so präziser lässt sich ein Bearbeitungswerkzeug führen.

Um zusätzlich Bahnkurven für eine Translations-Schwenk-Bewegung in beliebiger Weise vorgeben zu können, kann vorteilhafterweise vorgesehen sein, dass ein Abstand zwischen dem ersten und dem zweiten Führungselement veränderbar ist.

Gemäß einer bevorzugten Ausführungsform der Erfindung kann vorgesehen sein, dass das erste und das zweite Führungselement relativ zueinander so angeordnet und aufeinander abgestimmt sind, dass ein distales Ende eines Bearbeitungswerkzeugs längs einer geraden oder im Wesentlichen geraden Linie parallel oder im Wesentlichen parallel zur Längsachse bewegbar ist. Diese Ausgestaltung gestattet es, mit Hilfe des Führungsinstruments und einem an diesem geführten Bearbeitungswerkzeug eine geradlinige Nut oder einen Rücksprung an einem Knochen auszubilden, beispielsweise an einem Wirbelkörper.

Um das Führungsinstrument in definierter Weise an einen Wirbelkörper anlegen zu können, insbesondere zum Präparieren desselben vor dem Einsetzen eines Zwischenwirbelimplantats in einen Zwischenwirbelraum zwischen benachbarte Wirbelkörper, ist es günstig, wenn am distalen Ende des Instrumentenkörpers ein Implantatkörper zum Einführen in den Zwischenwirbelraum zwischen benachbarte Wirbelkörper einer menschlichen oder tierischen Wirbelsäule vorgesehen ist und wenn der Implantatkörper zwei an die Wirbelkörper anlegbare Anlageflächen aufweist. Insbesondere ist es so möglich, den Implantatkörper in den Zwischenwirbelraum einzuführen und mit einem am Führungsinstrument zwangsgeführten Bearbeitungswerkzeug einen oder beide benachbarte Wirbelkörper in gewünschter Weise zu bearbeiten.

Um das Führungsinstrument besonders leicht reinigen zu können, ist es günstig, wenn der Implantatkörper mit dem Instrumentenkörper lösbar verbindbar ist. Dies hat zudem den Vorteil, dass mit dem Instrumenterikörper unterschiedliche Implantatkörper verbunden werden können, so dass das Führungsinstrument universell für die gesamte Wirbelsäule verwendbar ist.

Damit benachbarte Wirbelkörper zum Bearbeiten derselben in einer gewünschten natürlichen Stellung gehalten werden können, ist es vorteilhaft, wenn die Anlageflächen relativ zueinander um einen Neigungswinkel geneigt sind. So können die benachbarten Wirbelkörper in einer Stellung bearbeitet werden, die derjenigen nach Einsetzen eines Zwischenwirbelimplantats entspricht.

Gemäß einer bevorzugten Ausführungsform der Erfindung kann vorgesehen sein, dass ein Satz Implantatkörper vorgesehen ist und dass die Implantatkörper des Satzes eine unterschiedliche Größe und/oder einen unterschiedlichen Neigungswinkel der Anlageflächen relativ zueinander und/oder einen unterschiedlichen Abstand der Anlageflächen voneinander aufweisen. Ein solcher Implantatkörpersatz macht es möglich, mit nur einem Instrumentenkörper auszukommen und das Führungsinstrument trotzdem zur Vorbereitung unterschiedlicher Patienten und unterschiedlicher Zwischenwirbelräume einsetzen zu können.

Eine zusätzliche Führung für das Bearbeitungswerkzeug kann auf einfache Weise dadurch vorgesehen werden, dass am Implantatkörper mindestens eine Führungsnut in einer der beiden Anlageflächen parallel oder im Wesentlichen parallel zur Längsachse des Instrumentenkörpers verlaufend angeordnet ist. Insbesondere können beide Anlageflächen mit Führungsnuten versehen sein.

Damit das Führungsinstrument nicht zu tief in einen menschlichen oder tierischen Körper, insbesondere in einen Zwischenwirbelraum desselben, eingeführt werden kann, ist es vorteilhaft, wenn eine Anschlagvorrichtung zum Begrenzen einer Einführtiefe des Implantatkörpers in den Zwischenwirbelraum vorgesehen ist.

Eine besonders einfach zu bedienende und herzustellende Anschlagvorrichtung umfasst mindestens einen quer und im Wesentlichen quer zur Längsachse des Instrumentenkörpers abstehenden Vorsprung. Ein solcher Vorsprung verhindert, dass das Instrument, sobald der Vorsprung am Wirbelkörper anliegt oder anschlägt, weiter in Richtung auf den Wirbelkörper hin bewegt werden kann.

Vorzugsweise ist die Anschlagvorrichtung mit dem Implantatkörper oder mit dem Instrumentenkörper lösbar verbindbar. Dies ermöglicht es, die Anschlagvorrichtung beispielsweise dann zu entfernen, wenn sie entweder nicht benötigt wird oder die Sicht auf den Operationsbereich durch die Anschlagvorrichtung besonders stark eingeschränkt wäre. Zudem lässt sich das Führungsinstrument so zu Reinigungszwecken auf einfache Weise zerlegen.

Ein besonders einfacher Aufbau der Anschlagvorrichtung wird dadurch erreicht, dass diese einen Träger umfasst und dass der Implantatkörper oder der Instrumentenkörper eine Führungseinrichtung für den Träger umfasst zum Führen einer Bewegung des Trägers relativ zum Instrumentenkörper und/oder zum Implantatkörper parallel oder im Wesentlichen parallel zur Längsachse.

Um die Anschlagvorrichtung oder Teile derselben in definierter Weise bewegen zu können, kann vorteilhafterweise vorgesehen sein, dass die Führungsvorrichtung mindestens einen am Implantatkörper in proximaler Richtung abstehenden Führungsstab umfasst und dass der Träger auf dem mindestens einen Führungsstab verschiebbar gelagert ist.

Damit eine Stellung der Anschlagvorrichtung in definierter Weise vorgebbar ist, ist es günstig, wenn die Anschlagvorrichtung ein Anschlagglied umfasst und wenn ein Abstand zwischen dem Träger veränderbar ist.

Der mindestens eine Vorsprung lässt sich auf einfache Weise relativ zum Führungsinstrument und/oder zum Implantatkörper bewegen und kann damit eine Einführtiefe für das Führungsinstrument vorgeben, wenn der Träger den mindestens einen Vorsprung trägt.

Das Führungsinstrument lässt sich ferner besonders gut halten, wenn am proximalen Ende des Instrumentenkörpers ein Griffteil vorgesehen ist.

Um es einem Operateur zu ermöglichen, von ihm bevorzugte Griffteile zu verwenden, ist es vorteilhaft, wenn der Griffteil mit dem Instrumentenkörper lösbar verbindbar ist. Ferner lässt sich so das Instrument auf einfache Weise sehr gut reinigen.

Um die Sicht auf den Operationsbereich nicht zusätzlich zu beeinträchtigen, ist es günstig, wenn der Griffteil und der Instrumentenkörper relativ zueinander abgewinkelt sind.

Sowohl die Konstruktion als auch die Herstellung des Führungsinstruments vereinfachen sich, wenn dieses spiegelsymmetrisch zu einer die Längsachse enthaltenden Spiegelebene ausgebildet ist.

Die eingangs gestellte Aufgabe wird ferner bei einem chirurgischen Instrumentarium, umfassend mindestens ein chirurgisches, zum Bearbeiten von Wirbelkörpern ausgebildetes und ein distales Werkzeugende aufweisendes Bearbeitungswerkzeug und mindestens ein chirurgisches Führungsinstrument für das mindestens eine chirurgische Bearbeitungswerkzeug, wobei das Führungsinstrument einen im Wesentlichen langgestreckten, ein proximales und ein distales Ende aufweisenden und eine Längsachse definierenden Instrumentenkörper umfasst, der eine Führungsvorrichtung für das Bearbeitungswerkzeug trägt, wobei die Führungsvorrichtung derart angeordnet und ausgebildet ist, dass mit ihr das Bearbeitungswerkzeug entlang einer von der Führungsvorrichtung definierten Bewegungsbahn zwangsführbar ist und dass die Bewegungsbahn einer überlagerten Translations-Schwenk-Bewegung entspricht, erfindungsgemäß dadurch gelöst, dass die Führungsvorrichtung ein erstes Führungselement umfasst, welches eine Schwenkachse für das Bearbeitungswerkzeug definiert, so dass das Bearbeitungswerkzeug relativ zum ersten Führungselement verschiebbar und um die Schwenkachse verschwenkbar ist.

Mit einem derartigen Instrumentarium lassen sich Teile des menschlichen Körpers, beispielsweise Knochen, insbesondere Wirbelkörper, mit einem Bearbeitungswerkzeug auf gewünschte Weise einfach und sicher bearbeiten.

Vorzugsweise ist das chirurgische Führungsinstrument des Instrumentariums eines der oben beschriebenen Instrumente.

Ein Knochen, insbesondere ein Wirbelkörper, lässt sich besonders einfach bearbeiten, wenn das mindestens eine Bearbeitungswerkzeug ein Fräswerkzeug mit einem langgestreckten Schaft ist und wenn das distale Werkzeugende ein rotierender Fräskopf ist. Ein derartiges Bearbeitungswerkzeug lässt sich mit dem Führungsinstrument einfach und sicher zwangsführen und so eine definierte Ausnehmung in einem Knochen präparieren.

Die Erfindung wird in den Ansprüchen definiert.

Die nachfolgende Beschreibung einer bevorzugten Ausführungsform der Erfindung dient im Zusammenhang mit der Zeichnung der näheren Erläuterung. Es zeigen:
- Figur 1 :: eine perspektivische Ansicht eines erfindungsgemäßen Instrumentariums beim Präparieren eines Wirbelkörpers;
- Figur 2:: eine perspektivische Gesamtansicht des erfindungsgemäßen Instrumentariums aus Figur 1;
- Figur3:: eine vergrößerte, teilweise geschnittene Ansicht eines distalen Endes des erfindungsgemäßen Instrumentariums;
- Figur 4:: eine vergrößerte perspektivische Ansicht eines proximalen Endes des erfindungsgemäßen Instrumentariums; und
- Figur 5:: eine teilweise geschnittene Seitenansicht des proximalen Endes des erfindungsgemäßen Instrumentariums.

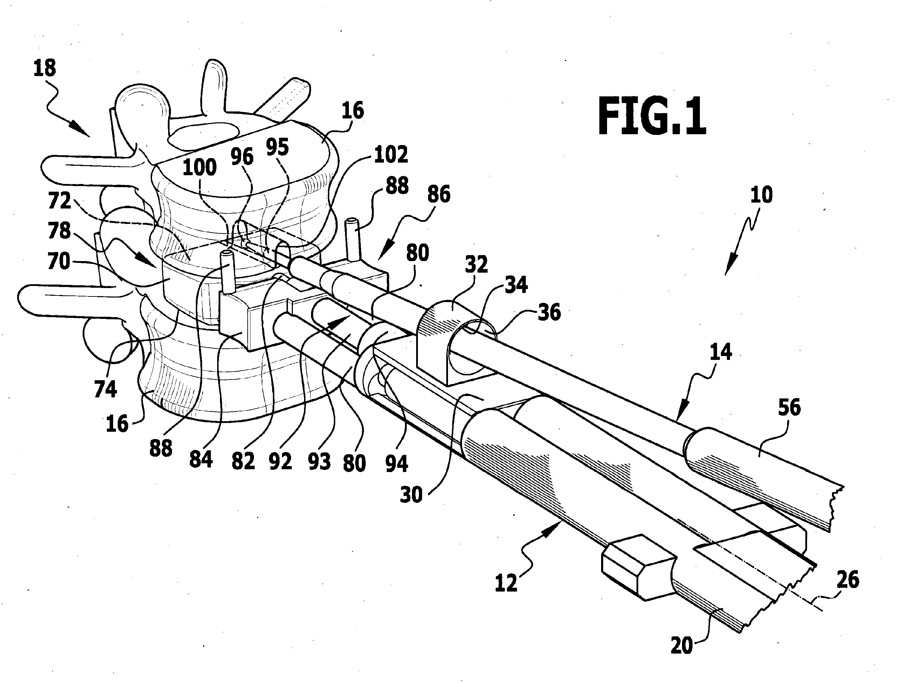

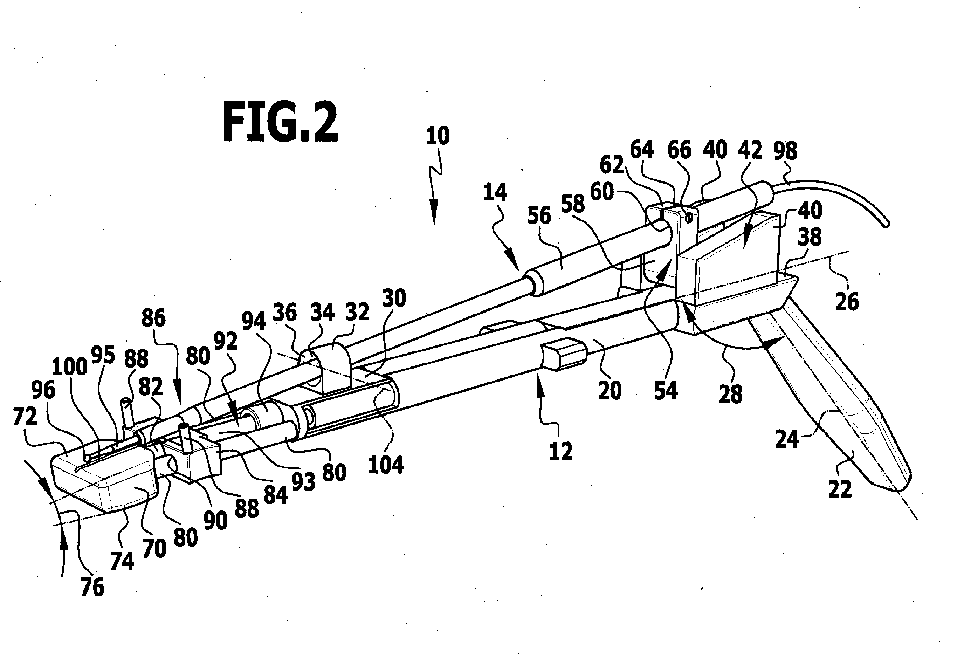

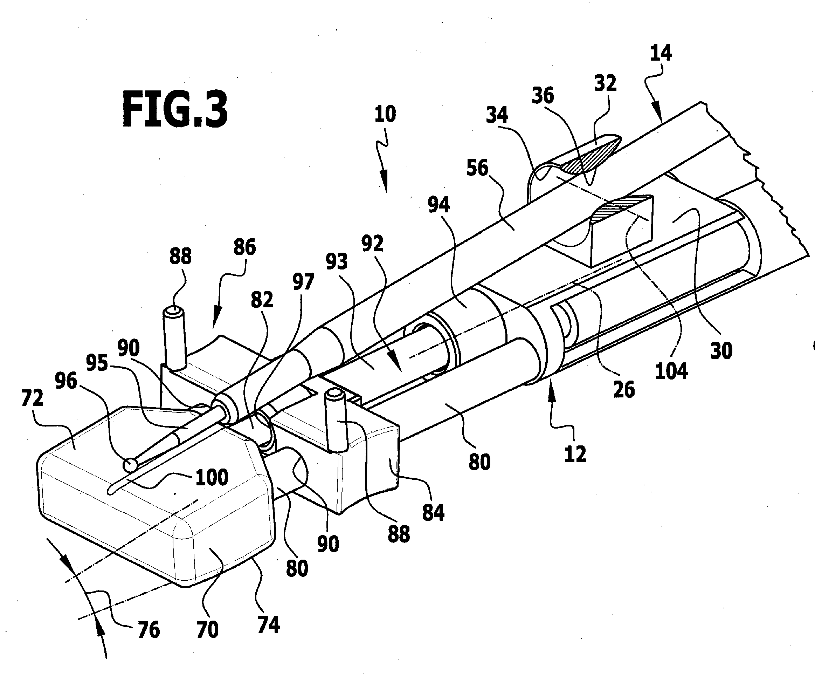

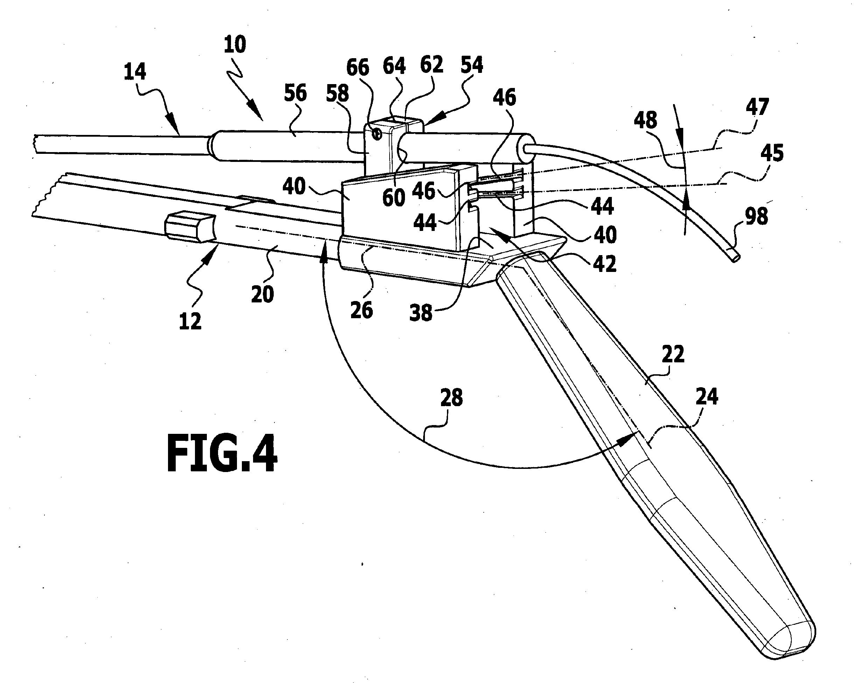

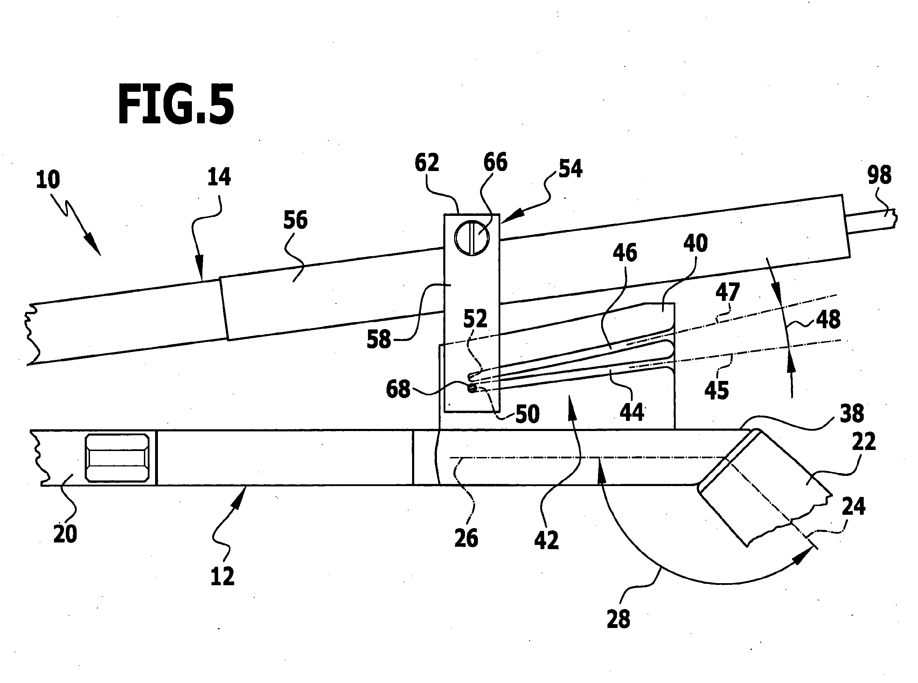

Das in den Figuren 1 bis 5 dargestellte und insgesamt mit den Bezugszeichen 10 versehene Instrumentarium, umfassend ein chirurgisches Führungsinstrument 12 und ein in Form eines Fräsers 14 ausgebildetes chirurgisches Bearbeitungswerkzeug 14, dient unter anderem, wie in Figur 1 dargestellt, zur Bearbeitung von Wirbelkörpern 16 einer menschlichen oder tierischen Wirbelsäule 18, wie nachfolgend näher erläutert wird.

Das Führungsinstrument 12 umfasst einen langgestreckten, ein proximales und ein distales Ende aufweisenden Instrumentenkörper 20. Vom proximalen Ende weg erstreckt sich ein Handgriff 22, dessen Längsachse 24 mit einer Längsachse 26 des Instrumentenkörpers 20 einen stumpfen Winkel 28 von etwa 135° einschließt. Im Bereich des distalen Endes des Instrumentenkörpers 20 ist ein im Wesentlichen quaderförmiges Tragelement 30 ausgebildet, welches ein erstes Führungselement 32 in Form einer Führungshülse trägt. Diese umfasst eine im Wesentlichen parallel zur Längsachse 26 verlaufende Durchbrechung 34, die eine in Richtung auf eine Symmetrieachse der Durchbrechung 34 hin weisende konvex gekrümmte Führungsfläche 36 definiert. Bei dem in den Figuren dargestellten Führungsinstrument 12 ist das Führungselement 32 relativ zum Instrumentenkörper 20 unbeweglich. Denkbar wäre es jedoch auch, das Führungselement 32 am Instrumentenkörper 20 beweglich anzuordnen, beispielsweise parallel zur Längsachse 26 verschiebbar. Auch wäre es möglich, das Führungselement 32 in einer Ebene, die zur Längsachse 26 senkrecht orientiert ist, verschiebbar oder verschwenkbar am Instrumentenkörper 20 zu lagern.

Im Bereich des proximalen Endes des Instrumentenkörpers 20 ist eine im Wesentlichen parallel zum Tragelement 30 angeordnete Plattform 38 vorgesehen, die zwei symmetrisch angeordnete trapezförmige Führungsglieder in Form von Lagerböcken 40 trägt, die gemeinsam ein zweites Führungselement 42 bilden. Jeder Lagerbock 40 umfasst zwei Führungsnuten 44 und 46, deren Längsachsen 45 und 47 sowohl relativ zueinander geneigt sind und einen Neigungswinkel 48 einschließen als auch relativ zur Längsachse 26 geneigt sind. Die Führungsnuten 44 und 46 verlaufen geradlinig und sind derart in den Lagerböcken 40 ausgebildet, dass sie in Richtung auf den jeweils spiegelsymmetrisch zu einer die Längsachse 26 enthaltenden Symmetrieebene angeordneten Lagerbock 40 hin weisen. Ein proximales Ende der Führungsnuten 44 und 46 ist geöffnet, ein distales Ende derselben ist geschlossen und bildet jeweils einen Anschlag 50 bzw. 52.

Das Führungsinstrument 12 weist ferner eine Halterung 54 zum klemmenden Halten eines Schafts 56 des Fräsers 14 auf. Die Halterung 54 umfasst einen im Wesentlichen quaderförmigen Haltekörper 58, der mit einer Bohrung 60 sowie einem eine Stirnseite 62 mit der Bohrung 60 verbindenden Schlitz 64 versehen ist. Die insgesamt schellenartige Halterung 54 umfasst ferner eine Klemmschraube 66, die in einer mit einem Innengewinde versehenen, quer zum Schlitz 64 angeordneten Bohrung angeordnet ist und die durch den Schlitz 64 getrennten freien Enden des Haltekörpers 58 miteinander verbindet und zusammenziehen kann, so dass ein Durchmesser der Bohrung 60 etwas verringerbar ist, um den Schaft 56 des Fräsers 14 klemmend, in der Bohrung 60 zu halten.

Am Haltekörper 58 sind im Wesentlichen parallel zur Klemmschraube 66 seitlich in entgegengesetzten Richtungen abstehende Lagerzapfen 68 angeordnet, die mit den erste Führungsglieder bildenden Führungsnuten 44 und 46 zusammenwirkende zweite Führungsglieder bilden. Die Lagerzapfen 68 sind so ausgebildet, dass sie von proximal her kommend in die Führungsnuten 44 und 46 einführbar und in diesen verschiebbar sind, so dass die Halterung 54 insgesamt relativ zum Instrumentenkörper 20 längs den durch die Führungsnuten 44 und 46 gebildeten Führungsbahnen verschiebbar gelagert ist. Damit kann der an der Halterung 54 klemmend gehaltene Fräser 14 in definierter Weise relativ zum Instrumentenkörper 20 bewegt und an diesem gelagert werden.

Das Führungsinstrument 12 umfasst ferner einen Implantatkörper 70, welcher zwei Anlageflächen 72 und 74 aufweist, die relativ zueinander um einen Neigungswinkel 76 geneigt sind. Der Implantatkörper 70 ist derart ausgebildet, dass er in einen Zwischenwirbelraum 78 zwischen zwei benachbarte Wirbelkörper 16 eingeführt werden kann. Vorzugsweise ist ein Satz von Implantatkörpern 70 vorgesehen, die in Form und Größe variieren sowie unterschiedliche Neigungswinkel 76 zwischen den Anlageflächen 72 und 74 aufweisen. Dadurch können sie in jeden beliebigen Zwischenwirbelraum der Wirbelsäule 18, die alle unterschiedlich groß sind und bei denen von den Wirbelkörpern 16 definierte Flächen relativ zueinander unterschiedlich stark geneigt sind, optimal eingeführt werden, um den ursprünglich mit einer Bandscheibe ausgefüllten Zwischenwirbelraum 78 optimal auszufüllen und die benachbarten Wirbelkörper 16 gegeneinander während des chirurgischen Eingriffs abzustützen.

Am Implantatkörper 70 sind zwei in proximaler Richtung weisend abstehende, parallel zueinander verlaufende Haltestäbe 80 angeordnet, die in nicht näher dargestellter Weise mit dem Tragelement 30 lösbar verbindbar sind. Dies ermöglicht es, den Instrumentenkörper 20 je nach Bedarf mit einem jeweils für den zu bearbeitenden Zwischenwirbelraum 78 optimal geeigneten Implantatkörper 70 zu verbinden.

Zwischen den Haltestäben 80 erstreckt sich parallel zu diesen ein kurzer Kupplungszapfen 82, der mit einer Schraubenspindel 92 einer insgesamt mit dem Bezugszeichen 86 versehenen Anschlagvorrichtung lösbar verbindbar ist. Zwei quer zur Längsachse 26 Von einem quaderförmigen Träger 84, der sich quer zur Längsachse erstreckt, stehen zwei zylindrische Vorsprünge 88 quer zur Längsachse 26 und quer zum Träger 84 ab und bilden Anschläge für die Wirbelkörper 16. Der Träger 84 ist mit zwei Bohrungen 90 versehen, durch die die Haltestäbe 80 durchgesteckt werden können, so dass der Träger 84 auf diesen verschiebbar gelagert ist.

Ein Abstand zwischen dem Träger 84 und dem Implantatkörper 70 lässt sich mittels der Schraubenspindel 92 variieren, die einen sich in Richtung der Längsachse 26 erstreckenden, mit einem Außengewinde versehenen Gewindeabschnitt 93 und einen in proximaler Richtung weisenden, ein Betätigungsglied bildenden Schraubenkopf 94 umfasst. Am Träger 84 ist eine zwischen den Haltestäben 80 und parallel zu diesen verlaufende, mit einem Innengewinde versehene Bohrung angeordnet, in die der Gewindeabschnitt 93 eingeschraubt ist. Der Gewindeabschnitt 93 ist an seinem distalen Ende mit einem Sackloch 97 versehen, welches ein Drehlager zur Aufnahme des Kupplungszapfens 82 bildet. Die Schraubenspindel 92 ist somit auf dem Kupplungszapfen 82 gelagert und am Träger 84 gehalten. Durch Drehen der zwischen dem Tragelement 30 und dem Kupplungszapfen in axialer Richtung unbeweglich gehaltenen Schraubenspindel 92 kann der Träger auf den Haltestäben parallel zur Längsachse 26 in distaler und proximaler Richtung bewegt werden. Der Träger 84 kann in proximaler Richtung bewegt werden, bis er an den Schraubenkopf 94 anschlägt, in distaler Richtung, bis er an den Implantatkörper 70 anschlägt. Ein maximaler Abstand zwischen dem Träger 84 und dem Implantatkörper 70 wird definiert durch die Position des Trägers 84, in der er am Schraubenkopf 94 anschlägt.

Der Fräser 14 weist eine im Schaft 56 rotierbar gelagerte Welle auf, die distalseitig in einem kugelförmigen Fräskopf 96 endet. Der Schaft 56 verjüngt sich ausgehend von seinem proximalen Ende in Richtung auf sein distales Ende hin in mehreren Stufen. Ferner wird der Fräser durch einen nicht näher dargestellten Antrieb angetrieben, der beispielsweise über ein Anschlusskabel 98 mit Strom versorgt werden kann. Alternativ wäre es auch denkbar, anstelle des Anschlusskabels 98 eine rotierbare, flexible Antriebswelle zum Antreiben der Welle 95 vorzusehen.

Die Verwendung des Instrumentariums 10 zum Präparieren der Wirbelkörper 16 wird nachfolgend näher beschrieben.

Zur Vorbereitung des Eingriffs wird das Führungsinstrument 12 vorbereitet. Hierzu wird ein Implantatkörper 70 ausgewählt und mit dem Instrumentenkörper 20 verbunden, der den Zwischenwirbelraum 78 zwischen den zu bearbeitenden Wirbelkörpern 16 in gewünschter Weise ausfüllt. Vorzugsweise weist der Implantatkörper 70 eine sich parallel oder im Wesentlichen parallel zur Längsachse 26 erstreckende Längsnut 100 in einer oder beiden Anlageflächen 72 beziehungsweise 74 auf. Das Anschlagglied 92 wird so eingestellt, dass die Vorsprünge 78 ihre distalste Stellung einnehmen, das heißt der Träger 84 nimmt dann ebenfalls seine distalste Stellung ein, in welcher ein Abstand zwischen dem Implantatkörper 70 und dem Träger 84 minimal ist.

Ein zur Bearbeitung der Wirbelkörper 16 vorgesehenes Bearbeitungswerkzeug, beispielsweise der Fräser 14, wird mit seinem Schaft 56 durch die Bohrung 60 der Halterung 54 geschoben und mittels der Klemmschraube 66 klemmend mit der Halterung 54 verbunden. Der so vorbereitete Fräser 14 wird mit seinem distalen Ende voran, also mit dem Fräskopf 96, durch die Durchbrechung 34 des Führungselements 32 hindurchgeschoben. Ein minimaler Durchmesser der Durchbrechung 34 ist nur unwesentlich größer als ein Außendurchmesser des im Bereich des ersten Führungselements 32 geführten Abschnitt des Schafts 56. Die Lagerzapfen 68 werden dann jeweils in eine der Nuten 44 oder 46 von proximal her kommend eingeschoben, wodurch dann der Fräser 14 definiert relativ zum Führungsinstrument 12 bewegbar ist. Wird der Fräser 14 in distaler Richtung bewegt, so wird dieser längs einer Bewegungsbahn zwangsgeführt; die einer überlagerten Translations-Schwenk-Bewegung entspricht. Der Schaft 56 des Fräsers 14 gleitet somit einerseits translatorisch im ersten Führungselement 32, andererseits ist diese Translationsbewegung von einer Schwenk- oder Abrollbewegung an der konvex gekrümmten Führungsfläche 36 überlagert. Das Führungselement 32 definiert auf Grund seiner Ausbildung eine Schwenkachse 104, um die der gesamte Fräser 14 verschwenkt wird. Damit führt aber auch der Fräskopf 96 eine überlagerte Translations-Schwenk-Bewegung aus. Allerdings können die Führungsnuten 44 beziehungsweise 46 so ausgebildet sein, dass ein Zentrum des Fräskopfs 96 einer geradlinigen Bahn folgt. Somit folgt auch der kugelige Fräskopf 96 einer insgesamt geradlinigen Bahn. Selbstverständlich wäre es auch denkbar, die Führungsnuten 44 beziehungsweise 46 so auszubilden, dass ein Zentrum des Fräskopfs 96 und damit der Fräskopf 96 selbst einer einfach und/oder mehrfach gekrümmten Bahn folgt.

Zur Bearbeitung eines oder beider Wirbelkörper 16 wird vor dem Einführen des Fräsers 14 durch das Führungselement 32 der mit dem Instrumentenkörper 20 verbundene Implantatkörper 70 in den Zwischenwirbelkörper 78 eingeführt, bis die Vorsprünge 88 an einem der Wirbelkörper 16 anschlagen. Anschließend wird die Anschlagvorrichtung 86 so eingestellt, dass der Implantatkörper 70 bis zur gewünschten Einführtiefe in den Zwischenwirbelraum 78 eingeführt werden kann, wobei eine Bewegung des Führungsinstruments 12 in distaler Richtung wiederum durch die Vorsprünge 88 begrenzt wird, die an einem Wirbelkörper 16 anschlagen. Nimmt das Führungsinstrument 12 schließlich seine gewünschte Position ein, wird der Fräser 14 wie oben beschrieben mit dem Führungsinstrument 12 beweglich verbunden. Zum Einarbeiten einer Nut 102 in den Wirbelkörper 16 wird der Fräskopf 96 in Rotation versetzt und der Fräser 14 in distaler Richtung bewegt. Auf diese Weise wird in einer auf eine der Anlageflächen 72 bzw. 74 hin weisenden Fläche des Wirbelkörper 16 die Nut 102 eingearbeitet, die zur Aufnahme eines finnenartigen Vorsprungs an einem nicht dargestellten Zwischenwirbelimpläntat dient, welches nach Entfernen des Führungsinstruments 12 in den Zwischenwirbelraum 78 eingesetzt werden kann.

Durch die insgesamt etwas geneigte Lagerung des Fräsers 14 am Führungsinstrument 12 wird die Sicht auf den Operationsbereich deutlich verbessert, ebenfalls auch durch den seitlich am Instrumentenkörper 20 abstehenden Handgriff 22.

## Patentansprüche

1. Chirurgisches Führungsinstrument (12) für ein chirurgisches, zum Bearbeiten von Wirbelkörpern (16) ausgebildetes und ein distales Werkzeugende (96) aufweisendes Bearbeitungswerkzeug (14), wobei das Führungsinstrument (12) einen im Wesentlichen langgestreckten, ein proximales und ein distales Ende aufweisenden und eine Längsachse (26) definierenden Instrumentenkörper (20) umfasst, der eine Führungsvorrichtung (32, 42, 54) für das Bearbeitungswerkzeug (14) trägt, wobei die Führungsvorrichtung (32, 42, 54) derart angeordnet und ausgebildet ist, dass mit ihr das Bearbeitungswerkzeug (14) entlang einer von der Führungsvorrichtung (32, 42, 54) definierten Bewegungsbahn zwangsführbar ist und dass die Bewegungsbahn einer überlagerten Translations-Schwenk-Bewegung entspricht, **dadurch gekennzeichnet, dass** die Führungsvorrichtung (32, 42, 54) ein erstes Führungselement (32) umfasst, welches eine Schwenkachse (104) für das Bearbeitungswerkzeug (14) definiert, so dass das Bearbeitungswerkzeug (14) relativ zum ersten Führungselement (32) verschiebbar und um die Schwenkachse (104) verschwenkbar ist.

2. Führungsinstrument nach Anspruch 1, **dadurch gekennzeichnet, dass** die Führungsvorrichtung (32, 42, 54) derart angeordnet und ausgebildet ist, dass mit ihr eine Spitze oder ein Zentrum des distalen Werkzeugendes (96) entlang einer linearen Bahnkurve zwangsführbar ist.

3. Führungsinstrument nach einem der voranstehenden Ansprüche, **dadurch gekennzeichnet, dass** die Führungsvorrichtung (32, 42, 54) eine Halterung (54) für das Bearbeitungswerkzeug (14) umfasst.

4. Führungsinstrument nach Anspruch 3, **dadurch gekennzeichnet, dass** die Halterung (54) mit dem Bearbeitungswerkzeug (14) lösbar verbindbar ist.

5. Führungsinstrument nach einem der voranstehenden Ansprüche, **dadurch gekennzeichnet, dass** die Führungsvorrichtung (32, 42, 54) ein zweites Führungselement (42) umfasst zum Halten und Führen des Bearbeitungswerkzeugs (14) am Instrumentenkörper (20) und dass das Bearbeitungswerkzeug (14) relativ zum zweiten Führungselement (42) verschiebbar und verschwenkbar lagerbar ist.

6. Führungsinstrument nach Anspruch 5, **dadurch gekennzeichnet, dass** das Bearbeitungswerkzeug (14) oder ein Teil (56) desselben am zweiten Führungselement (42) verschiebbar und um die Schwenkachse (104) verschwenkbar lagerbar ist.

7. Führungsinstrument nach einem der Ansprüche 5 oder 6, **dadurch gekennzeichnet, dass** das zweite Führungselement (42) mindestens ein erstes Führungsglied (44, 46) umfasst zum Führen des Bearbeitungswerkzeugs (14) oder eines Teil (56) desselben längs einer durch das zweite Führungselement (42) definierten Kurve.

8. Führungsinstrument nach Anspruch 7, **dadurch gekennzeichnet, dass** das zweite Führungselement (42) mindestens einen Führungskörper (40) umfasst und dass das mindestens eine erste Führungsglied (44, 46) in Form einer am mindestens einen Führungskörper (40) angeordneten Führungsnut ausgebildet ist.

9. Führungsinstrument nach Anspruch 8, **dadurch gekennzeichnet, dass** die Halterung (54) mindestens ein zweites Führungsglied (68) umfasst und dass das mindestens eine zweite Führungsglied (68) derart ausgebildet ist, dass es am ersten Führungsglied (44, 46) bewegbar gelagert ist.

10. Führungsinstrument nach Anspruch 9, **dadurch gekennzeichnet, dass** das mindestens eine zweite Führungsglied (68) ein quer oder im Wesentlichen quer zur Längsachse (26) abstehender, zum Eintauchen in die mindestens eine Führungsnut (44, 46) geformter Führungsvorsprung (68) ist.

11. Führungsinstrument nach einem der voranstehenden Ansprüche, **dadurch gekennzeichnet, dass** am distalen Ende des Instrumentenkörpers (20) ein Implantatkörper (70) zum Einführen in einen Zwischenwirbelraum (78) zwischen benachbarte Wirbelkörper (16) einer menschlichen oder tierischen Wirbelsäule (18) vorgesehen ist und dass der Implantatkörper (70) zwei an die Wirbelkörper (16) anlegbare Anlageflächen (72, 74) aufweist.

12. Führungsinstrument nach Anspruch 11, **dadurch gekennzeichnet, dass** der Implantatkörper (70) mit dem Instrumentenkörper (20) lösbar verbindbar ist.

13. Führungsinstrument nach Anspruch 11 oder 12, **dadurch gekennzeichnet, dass** ein Satz Implantatkörper (70) vorgesehen ist und dass die Implantatkörper (70) des Satzes eine unterschiedliche Größe und/oder einen unterschiedlichen Neigungswinkel (76) der Anlageflächen (72, 74) relativ zueinander und/oder einen unterschiedlichen Abstand der Anlageflächen (72, 74) voneinander aufweisen.

14. Chirurgisches Instrumentarium (10) umfassend mindestens ein chirurgisches, zum Bearbeiten von Wirbelkörpern (16) ausgebildetes und ein distales Werkzeugende (96) aufweisendes Bearbeitungswerkzeug (14) und mindestens ein chirurgisches Führungsinstrument (12) für das mindestens eine chirurgische Bearbeitungswerkzeug (14), wobei das Führungsinstrument (12) einen im Wesentlichen langgestreckten, ein proximales und ein distales Ende aufweisenden und eine Längsachse (26) definierenden Instrumentenkörper (20) umfasst, der eine Führungsvorrichtung (32, 42, 54) für das Bearbeitungswerkzeug (14) trägt, wobei die Führungsvorrichtung (32, 42, 54) derart angeordnet und ausgebildet ist, dass mit ihr das Bearbeitungswerkzeug (14) entlang einer von der Führungsvorrichtung (32, 42, 54) definierten Bewegungsbahn zwangsführbar ist und dass die Bewegungsbahn einer überlagerten Translations-Schwenk-Bewegung entspricht, **dadurch gekennzeichnet, dass** die Führungsvorrichtung (32, 42, 54) ein erstes Führungselement (32) umfasst, welches eine Schwenkachse (104) für das Bearbeitungswerkzeug (14) definiert, so dass das Bearbeitungswerkzeug (14) relativ zum ersten Führungselement (32) verschiebbar und um die Schwenkachse (104) verschwenkbar ist.

15. Instrumentarium nach Anspruch 14, **dadurch gekennzeichnet, dass** das mindestens eine chirurgische Führungsinstrument (12) ein Instrument (12) nach einem der Ansprüche 2 bis 13 ist.

## Claims

1. Surgical guiding instrument (12) for a surgical machining tool (14) which is configured for machining vertebral bodies (16) and has a distal tool end (96), the guiding instrument (12) comprising a substantially elongated instrument body (20) having a proximal and a distal end and defining a longitudinal axis (26), the instrument body (20) carrying a guiding device (32, 42, 54) for the machining tool (14), wherein the guiding device (32, 42, 54) is arranged and constructed so as to enable the machining tool (14) to be positively guided by it along a path of movement defined by the guiding device (32, 42, 54), and the path of movement corresponds to a superimposed translational-pivotal movement, **characterized in that** the guiding device (32, 42, 54) comprises a first guiding element (32) defining a pivot axis (104) for the machining tool (14), so that the machining tool (14) is displaceable relative to the first guiding element (32) and is pivotable about the pivot axis (104).

2. Guiding instrument in accordance with claim 1, **characterized in that** the guiding device (32, 42, 54) is arranged and constructed so as to enable a tip or a center of the distal tool end (96) to be positively guided by it along a linear path curve.

3. Guiding instrument in accordance with claim 1, **characterized in that** the guiding device (32, 42, 54) comprises a holder (54) for the machining tool (14).

4. Guiding instrument in accordance with claim 3, **characterized in that** the holder (54) is releasably connectable to the machining tool (14).

5. Guiding instrument in accordance with any one of the preceding claims, **characterized in that** the guiding device (32, 42, 54) comprises a second guiding element (42) for holding and guiding the machining tool (14) on the instrument body (20), and **in that** the machining tool (14) is mountable for sliding displacement and pivotal movement relative to the second guiding element (42).

6. Guiding instrument in accordance with claim 5, **characterized in that** the machining tool (14) or a part (56) thereof is mountable for sliding displacement on the second guiding element (42) and pivotal movement about the pivot axis (104).

7. Guiding instrument in accordance with any one of claims 5 or 6, **characterized in that** the second guiding element (42) comprises at least one first guiding member (44, 46) for guiding the machining tool (14) or a part (56) thereof along a curve defined by the second guiding element (42).

8. Guiding instrument in accordance with claim 7, **characterized in that** the second guiding element (42) comprises at least one guiding body (40), and the at least one first guiding member (44, 46) is in the form of a guide groove arranged on the at least one guiding body (40).

9. Guiding instrument in accordance with claim 8, **characterized in that** the holder (54) comprises at least one second guiding member (68), and the at least one second guiding member (68) is constructed so as to be movably mounted on the first guiding member (44, 46).

10. Guiding instrument in accordance with claim 9, **characterized in that** the at least one second guiding member (68) is a guiding projection (68) which protrudes transversely or substantially transversely to the longitudinal axis (26) and is shaped so as to enter into the at least one guide groove (44, 46).

11. Guiding instrument in accordance with any one of the preceding claims, **characterized in that** an implant body (70) for insertion into an intervertebral space (78) between adjacent vertebral bodies (16) of the vertebral column (18) of a human being or an animal is provided at the distal end of the instrument body (20), and **in that** the implant body (70) comprises two contact surfaces (72, 74) adapted for placement on the vertebral bodies (16).

12. Guiding instrument in accordance with claim 11, **characterized in that** the implant body (70) is releasably connectable to the instrument body (20).

13. Guiding instrument in accordance with any one of claims 11 or 12, **characterized in that** a set of implant bodies (70) is provided, and the implant bodies (70) of the set have a different size and/or a different angle of inclination (76) of the contact surfaces (72, 74) relative to each other and/or a different spacing of the contact surfaces (72, 74) from each other.

14. Surgical instrumentarium (10) comprising at least one surgical machining tool (14) which is configured for machining vertebral bodies (16) and has a distal tool end (96), and at least one surgical guiding instrument (12) for the at least one surgical machining tool (14), the guiding instrument (12) comprising a substantially elongated instrument body (20) having a proximal and a distal end and defining a longitudinal axis (26), and the instrument body (20) carrying a guiding device (32, 42, 54) for the machining tool (14), wherein the guiding device (32, 42, 54) is arranged and constructed so as to enable the machining tool (14) to be positively guided by it along a path of movement defined by the guiding device (32, 42, 54), and the path of movement corresponds to a superimposed translational-pivotal movement, **characterized in that** the guiding device (32, 42, 54) comprises a first guiding element (32) defining a pivot axis (104) for the machining tool (14), so that the machining tool (14) is displaceable relative to the first guiding element (32) and is pivotable about the pivot axis (104).

15. Instrumentarium in accordance with claim 14, **characterized in that** the at least one surgical guiding instrument (12) is an instrument (12) in accordance with any one of claims 2 to 13.

## Revendications

1. Instrument de guidage chirurgical (12), pour un outil d'usinage chirurgical (14) conçu pour l'usinage de corps de vertèbres (16) et présentant une extrémité d'outil distale (96), l'instrument de guidage (12) comportant un corps d'instrument (20) sensiblement allongé, qui présente une extrémité proximale et distale, définit un axe longitudinal (26) et porte un dispositif de guidage (32, 42, 54) pour l'outil d'usinage (14), le dispositif de guidage (32, 42, 54) étant agencé et réalisé de manière à ce qu'il permette d'assurer un guidage forcé de l'outil d'usinage (14) le long d'une trajectoire de mouvement définie par le dispositif de guidage (32, 42, 54), et de manière à ce que la trajectoire de mouvement corresponde à la superposition d'un mouvement de translation et de pivotement,
**caractérisé en ce que** le dispositif de guidage (32, 42, 54) comprend un premier élément de guidage (32), qui définit un axe de pivotement (104) pour l'outil d'usinage (14), de façon à ce que l'outil d'usinage (14) puisse coulisser par rapport au premier élément de guidage (32) et pivoter autour de l'axe de pivotement (104).

2. Instrument de guidage selon la revendication 1, **caractérisé en ce que** le dispositif de guidage (32, 42, 54) est agencé et conçu de manière telle, qu'il permette d'assurer le guidage forcé d'une pointe ou d'un centre de l'extrémité d'outil distale (96) le long d'une courbe de trajectoire, linéaire.

3. Instrument de guidage selon l'une des revendications précédentes, **caractérisé en ce que** le dispositif de guidage (32, 42, 54) comprend un système de support (54) pour l'outil d'usinage (14).

4. Instrument de guidage selon la revendication 3, **caractérisé en ce que** le système de support (54) peut être relié de manière amovible à l'outil d'usinage (14).

5. Instrument de guidage selon l'une des revendications précédentes, **caractérisé en ce que** le dispositif de guidage (32, 42, 54) comprend un deuxième élément de guidage (42) pour supporter et guider l'outil d'usinage (14) sur le corps d'instrument (20), et **en ce que** l'outil d'usinage (14) peut être monté de manière coulissante et pivotante par rapport au deuxième élément de guidage (42).

6. Instrument de guidage selon la revendication 5, **caractérisé en ce que** l'outil d'usinage (14) ou une partie (56) de celui-ci peut être monté sur le deuxième élément de guidage (42), de manière à pouvoir coulisser et pivoter autour de l'axe de pivotement (104).

7. Instrument de guidage selon l'une des revendications 5 ou 6, **caractérisé en ce que** le deuxième élément de guidage (42) comprend au moins un premier organe de guidage (44, 46) pour guider l'outil d'usinage (14) ou une partie (56) de celui-ci le long d'une courbe définie par le deuxième élément de guidage (42).

8. Instrument de guidage selon la revendication 7, **caractérisé en ce que** le deuxième élément de guidage (42) comprend au moins un corps de guidage (40), et **en ce que** ledit au moins un premier organe de guidage (44, 46) est réalisé sous la forme d'une rainure de guidage agencée sur ledit au moins un corps de guidage (40).

9. Instrument de guidage selon la revendication 8, **caractérisé en ce que** le système de support (54) comprend au moins un deuxième organe de guidage (68), et **en ce que** ledit au moins un deuxième organe de guidage (68) est conçu de façon à être monté de manière mobile sur le premier organe de guidage (44, 46).

10. Instrument de guidage selon la revendication 9, **caractérisé en ce que** ledit au moins un deuxième organe de guidage (68) est une protubérance de guidage (68) faisant saillie transversalement ou sensiblement de manière transversale à l'axe longitudinal (26), et configurée pour s'engager dans ladite au moins une rainure de guidage (44, 46).

11. Instrument de guidage selon l'une des revendications précédentes, **caractérisé en ce qu'**à l'extrémité distale du corps d'instrument (20) est prévu un corps d'implant (70) destiné à être introduit dans un espace intervertébral (78) entre des corps de vertèbres (16) voisins d'une colonne vertébrale (18) humaine ou animale, et **en ce que** le corps d'implant (70) présente deux surfaces d'appui (72, 74) pouvant être appliquées contre les corps de vertèbres (16).

12. Instrument de guidage selon la revendication 11, **caractérisé en ce que** le corps d'implant (70) peut être relié de manière amovible avec le corps d'instrument (20).

13. Instrument de guidage selon la revendication 11 ou la revendication 12, **caractérisé en ce qu'**il est prévu un jeu de corps d'implant (70), et **en ce que** les corps d'implant (70) du jeu présentent une grandeur différente et/ou un angle d'inclinaison (76) différent des surfaces d'appui (72, 74) l'une par rapport à l'autre et/ou une distance d'espacement différente entre les surfaces d'appui (72, 74).

14. Instrumentation chirurgicale (10) comprenant au moins un outil d'usinage chirurgical (14) conçu pour l'usinage de corps de vertèbres (16) et présentant une extrémité d'outil distale (96), et au moins un instrument de guidage chirurgical (12) pour ledit au moins un outil d'usinage chirurgical (14), l'instrument de guidage (12) comportant un corps d'instrument (20) sensiblement allongé, qui présente une extrémité proximale et distale, définit un axe longitudinal (26) et porte un dispositif de guidage (32, 42, 54) pour l'outil d'usinage (14), le dispositif de guidage (32, 42, 54) étant agencé et réalisé de manière à ce qu'il permette d'assurer un guidage forcé de l'outil d'usinage (14) le long d'une trajectoire de mouvement définie par le dispositif de guidage (32, 42, 54), et de manière à ce que la trajectoire de mouvement corresponde à la superposition d'un mouvement de translation et de pivotement,
**caractérisée en ce que** le dispositif de guidage (32, 42, 54) comprend un premier élément de guidage (32), qui définit un axe de pivotement (104) pour l'outil d'usinage (14), de façon à ce que l'outil d'usinage (14) puisse coulisser par rapport au premier élément de guidage (32) et pivoter autour de l'axe de pivotement (104).

15. Instrumentation selon la revendication 14, **caractérisée en ce que** ledit au moins un instrument de guidage chirurgical (12) est un instrument (12) selon l'une des revendications 2 à 13.
